# EUROPEAN PATENT APPLICATION

(11) **EP 2 030 604 A1**
(43) Date of publication of application: **04.03.2009**
(21) Application number: 07115061.9
(22) Date of filing: 28.08.2007
(51) Int. Cl.: A61K 6/10, A61C 9/00

(54) **Retraction of Sulcus**

(71) Applicant: Coltene Whaledent AG, 9450 Altstaetten (CH)
(72) Inventor: Kollefrath, Ralf, 9464, Ruethi (CH); Pittner, Brigitte, 6833, Klaus (AT); Mannschedel, Werner, 89129, Langenau (DE)
(74) Representative: Hepp, Dieter

(57) **Abstract**

The invention concerns a method of retraction of sulcus, in which, in a first step, a flowable retraction material (1) is applied onto the sulcus (5) of at least one tooth (4). In a second step, a dental dam (2) is applied onto the tooth (4) and brought into contact with or in the vicinity of the retraction material (1). According to the invention, the retraction material (1) and the material of the dental dam (2) are chosen such that they can form a chemical bond with each other. Moreover, the invention concerns a dental kit (9) for performing the method, which dental kit (9) contains at least one flowable retraction material (1) and at least one dental dam (2), wherein the retraction material (1) and the dental dam (2) can form a chemical bond. Additionally, the invention concerns the use of a dental kit (9) in the method. Finally, the invention concerns a dental retraction device (10) containing a retraction material (1) and a dental (2), which have formed a chemical bond with each other.

## Description

The invention concerns a method of retraction of sulcus, a dental kit for retraction of sulcus, a use of dental set for retraction of sulcus, and a dental retraction device.

For preparation of a dental restoration, especially dentures covering only a few teeth, an impression of the teeth and the part of the gingiva adjacent to these teeth must be provided to the dental technician. Therefore, a molding of the dental situation has to be prepared by the dentist. In order to cover the transition from the tooth to the jaw it is necessary to free the neck of the tooth which is covered by the gingiva. Thus for preparing the dental impression this area, the so-called sulcus, must be exposed.

In WO 2004/082510, there is disclosed a method of retraction of a sulcus in which, in a first step, a dental impression of a tooth is formed from a curable composition. Upon removal of the cured dental impression, an expandable silicone material is applied in the vicinity of the sulcus. In a further step, the impression is placed on the tooth again. This method is rather cumbersome since it involves many steps to be performed in the patient's mouth. Furthermore, the cured dental impression needs to be put aside temporarily.

EP 1 693 022 teaches the use of a cap pre-filled with a deformable material in order to direct an expandable retraction material into the sulcus. Therefore, this method entails the need of an additional part (the cap) and the further step of inserting the deformable material into the cap.

US 2007/0087304 discloses a method employing a dental dam consisting of a sponge, foam, rubber, or other type of porous, open or closed cellular material. During curing of the retraction material, this material is mechanically engaged within the pores of the dental dam. However, the use of a porous material in a dental dam has several disadvantages. On the one hand, the mechanical engagement is not very reliable. On the other hand, bacteria may be captured in the porous surface of the dental dam.

The object of the present invention is to avoid the disadvantages of the prior art, especially to provide an easier method for retraction of sulcus.

This object is solved by the method according to the independent claim. This method comprises the following steps:
- applying a flowable, preferably a curable retraction material onto the sulcus of at least one tooth and/or applying the said retraction material into a dental dam;
- applying a dental dam onto said at least one tooth; and
- allowing said dental dam to get in contact with said retraction material if the retraction material (1) has been applied onto the sulcus; or allowing said retraction material in the dental dam to get in contact with said sulcus.

According to the invention, the retraction material and the material of said dental dam are chosen such that they can form a chemical bond, preferably a covalent chemical bond, with each other.

The dental dam is suitable to form a chamber over at least a portion of at least one tooth and optionally over an adjacent outer part of a gingiva. Preferably, the dental dam is provided as a blank. Here and henceforth, the term "blank" refers to the property of the dental dam being preformed in a shape that is not individualized to the exact dental situation of a certain patient, as it is the case with an impression. Instead, the dental dam is only roughly adapted to the general shape and dimensions of a dental situation, e.g. as a straight or curved bar or rod.

Preferably, the dental dam is plastically deformable. More preferably, the dental dam has a Mooney viscosity ML(1+4/23 °C) of 1 - 300 MU, preferably of 10 - 250 MU, most preferably of 50 - 200 MU, according to DIN 53523-1960 (Normalrotor, 38,1 mm; however, deviating from DIN 53523-1960, the Mooney viscosity is measured at a temperature of 23 °C). This property sufficiently prevents the dam from an unwanted deformation, for example due to its own gravity and/or during storage. In particular, the requirement of using a separate cap is eliminated, in contrast to the prior art.

Preferably, the dental dam contains at least one silicone compound or a mixture of silicone compounds, preferably containing functional groups compatible with the retraction material (e.g. alkyl, alkenyl, alkynyl, hydroxyl or hydride groups). A dental dam containing a silicone compound and exhibiting a viscosity within the ranges given above is commonly denoted in the art as HTV (high-temperature vulcanizing) or "solid silicone". Modified siloxanes such as so-called bouncing putty or silly putty (siloxane chains comprising Si-O-B-O-Si bridges; cf. e.g. US 2,431,878) may also be used, as well as siloxane copolymers, e.g. comprising alkyl sidechains and/or urethane groups.

Preferably, the dental dam is thixotropic. This property further facilitates the application of the dental dam. The dental dam may be elastic. However, according to a preferable embodiment, the dental dam is plastically deformable, which allows it to be placed on the at least one tooth without any need to wait for a curing reaction of a separate impression material. Moreover, once a plastically deformable dental dam has been applied, it requires less force and is therefore more comfortable to hold it in place during the curing reaction of the material underneath, when the patient is asked to clench his/her teeth.

Preferably, the flowable retraction material has a viscosity between 1 and 100'000 mPas, preferably between 5 and 100 Pas, more preferably between 5 and 66 Pas. A retraction material endowed with such a viscosity can easily flow into the gap between a tooth and a sulcus.

According to one embodiment, the retraction material is an impression material. The retraction material may contain at least one silicone compound, which is preferably curable by an addition reaction. Preferably, the at least one silicone compound is crosslinkable by an addition reaction. The use of silicone materials crosslinkable by addition reactions avoids possible adverse effects to the health of the patients since during curing no harmful compounds are released.

Preferably, the retraction material is curable and can form a chemical bond with the dental dam during and/or after its curing. Curing reactions are to be understood as reaction methods which lead to the generation of new inter- and/or intramolecular bonds. Such a chemical bond permits the removal of the dental dam together with the retraction material, as will be outlined below. Preferably, the retraction material contains at least one silicone compound or a mixture of silicone compounds. Preferably, the retraction material contains a foaming agent. More preferably, at least one silicone compound of the retraction material contains at least one functional group crosslinkable with the functional groups of the dental dam, as outlined above (e.g. an alkyl, alkenyl, alkynyl, hydroxyl, or hydride group).

Preferably, at least a portion of the surface of the dental dam is substantially even. In particular, at least the portion of the surface which is suitable for a contact with a tooth and/or a gingiva (or tooth and/or gingiva covered with retraction material, respectively) does not exhibit any porosity. In contrast to US 2007/0087304, which requires a porous dental dam in order to form a mechanical bond, porosity of the surface of the dam according to the present invention is not mandatory due to the chemical nature of the bond. Therefore, the present method can prevent clogging with dirt in pores and the accumulation of bacteria; moreover, the even surface also permits a simplified cleaning of the surface of the dental dam.

According to a further embodiment, the retraction material expands, in particular during and/or after curing of the retraction material. Preferably, the retraction material exhibits a volume expansion of at least 20 %, preferably of at least 35 %, more preferably of at least 70 %. The values of this volume expansion are related to the expansion of the material in a non-limited, i. e. in an open volume. The expansion will usually start after the beginning of these reaction methods, i. e. with the mixing of the components, and will last beyond the end of these reactions.

Suitable silicone compounds which exhibit such an expansion behavior are silicone compounds crosslinkable by addition reactions disclosed in WO 2004/082510. An expanding silicone material exhibiting the above mentioned expansion volume allows an improved and simpler control of the sulcus retraction. Where appropriate, prior to the application of the silicone material, a curing catalyst is added or the silicone material includes already a catalyst which initiates the curing due to ambient humidity. In addition to the specific expansion behavior, a control can also be achieved due to the amount applied. Both factors thus mutually influence the result of the retraction and can be adjusted by routine measures by the person of skill in the art.

Advantageously, the retraction material, preferably an expandable addition crosslinkable silicone material, is used as a two-component system. Preferably, the components for the production of the retraction material are provided in a multi-chamber cartridge system, preferably in a two-chamber cartridge system. The application is performed by a mixing device, for which a static mixing device may be chosen.

For example, the different functionalized poly(dimethyl)siloxanes, such as dihydroxy- or divinyl- poly(dimethyl)siloxanes may be used as constituents of these two components, each possessing a viscosity preferably between 5 and 100 Pa.s. Both components may comprise additionally fillers which commonly are used for dental masses. These fillers may either be surface treated or be without any surface treatment. Examples for fillers are silica, pyrogeneous silica, calcium carbonate, milled quartz or silicates.

The method can be varied by applying at least one hemostatic compound or an astringent prior to the application of the retraction material. Examples of hemostatic compounds or astringents are - including the different, suitable hydrates - potassium aluminum sulfate, aluminum sulfate, aluminum iron sulfate, aluminum ammonium sulfate, iron chloride, aluminum chloride, sodium chloride, zinc chloride, zinc phenol sulfate, tannic acids, adrenalin or other known compounds. By applying these compounds, bleedings caused by the preparation of the dental situation prior to the application of the retraction material and/or the dental dam can be immediately stopped. Thus, after the removal of the extraction material, the displaced sulcus can easily be cleaned from leaked liquids and remains widely free of further leaking of liquids for a subsequent final molding of the dental situation. Since a chemical compatibility of the hemostatic compound with the retraction material, in particular with a silicone material, cannot be guaranteed at all times, both components have to be applied separately. But single hemostatic compounds like tannic acids can be incorporated into the addition crosslinkable, expanding silicone material, so that a common application to the area between tooth and gingiva is possible, if so desired.

Preferably, during the application of the dental dam, a chamber is formed over the retraction material. This chamber comprises as its walls at least a portion of one tooth, at least a portion of the dental dam, and an outer section of the gingiva or the retraction material on the gingiva. Such a chamber effectively directs the retraction material into the sulcus. Preferably, the dental dam exhibits a sufficient dimensional stability in order to direct the retraction material into the sulcus.

The dental dam may be covered by an impression tray or bite tray. Such an impression tray or bite tray prevents the dental dam from being bitten through by the patient when he/she is asked to clench his/her teeth. Moreover, the impression tray or bite tray may add to the dimensional stability when the viscosity of the dental dam requires it.

The dental dam and the retraction material may be jointly removed after the curing and/or the formation of the chemical bond. In particular, when the tear-off force induced by the chemical bond between the dental dam and the retraction material is sufficiently high, the cured and/or expanded retraction material may be removed from the tooth by gripping and pulling only the dental dam without any need to touch the retraction material itself.

In an alternative embodiment the cured retraction material is removed with the help of a cord, i.e. a retraction cord which has been embedded in the retraction material before the application of the dental dam. The cord is fixed within the retraction material during the curing method. The cord may also be placed in the area between the neck of the tooth and the gingiva prior to the application of the retraction material and then covered by the dental dam during the application. All materials suitable for dental application can be used as cords.

A further aspect of the invention is a dental kit which can be used for an application in the method disclosed above. The kit comprises at least one flowable, preferably a curable retraction material and at least one dental dam. The retraction material and the dental dam are chosen such that they can form a chemical bond with each other.

The dental dam is suitable to form a chamber over at least a portion of at least one tooth and optionally over an adjacent outer part of a gingiva. Preferably, the dam is in the form of a blank.

Advantageously, at least one dental dam of the kit has a Mooney viscosity ML(1+4/23 °C) of 1 - 300 MU, preferably of 10 - 250 MU, most preferably of 50 - 200 MU. This prevents the dam from an unwanted deformation, for example due to its own gravity and/or during storage. In particular, the requirement of using a separate cap is eliminated.

In a further embodiment, at least one dental dam of the kit is plastically deformable and can therefore be placed on the at least one tooth without any need to wait for a curing reaction of a separate impression material.

Moreover, the kit may contain a set of different dental dams having, for example, different materials, viscosities, deformability, or dimensions. This can enable the dentist to choose from the set a dental dam which is most appropriate for the particular treatment.

The retraction material of the dental kit may have one or more of the properties which were described above in connection with the method according to the invention. In particular, the retraction material may comprise at least one silicone compound, which is crosslinkable by an addition reaction. Moreover, the retraction material forms a chemical bond with said dental dam during and/or after curing. Preferably, the retraction material can expand during and/or after curing. Advantageously, a volume expansion of at least 20 %, preferably of at least 35 %, more preferably of at least 70 % occurs.

Preferably, at least one dental dam of the kit exhibits a sufficient dimensional stability in order to direct the retraction material into the sulcus.

More preferably, the chemical bond between the dental dam and the retraction material has a higher tear-off force than the bond between the retraction material and the tooth. This allows the joint removal of the dental dam and the cured retraction material and therefore simplifies the method. The cured and/or expanded retraction material may be removed from the tooth by gripping and pulling only the dental dam without touching the retraction material itself.

In a further embodiment of the dental kit, the retraction material comprises a hemostatic compound, preferably at least one tannic acid. In this case the mixture of tannic acid and expandable silicone material is applied to the area to be treated.

According to a further improvement, the dental kit also comprises an impression tray or bite tray for covering the dental dam. The application of an impression tray or bite tray can prevent the danger of biting through the dental dam when the patient is asked to clench his/her teeth. However, the impression tray or bite tray may also be omitted when the dental dam is chosen appropriately.

A further aspect of the invention concerns the use of a dental kit as disclosed above with one or more of the cited features in a method according to the invention.

Moreover, the invention concerns a dental retraction device for retracting a sulcus. The dental retraction device comprises a retraction material and a dental dam, wherein the retraction material and the dental dam have formed a chemical bond with each other. The retraction material and the dental dam of this dental retraction device may have one or more of the properties and advantages that are outlined above. In particular, a dental retraction device according to the invention can be obtained by a method of retraction of a sulcus according to the invention.

Finally, the invention concerns a dental dam having a Mooney viscosity ML(1+4/23 °C) of 1 - 300 MU, preferably of 10 - 250 MU, most preferably of 50 - 200 MU. The dental dam may furthermore have one or more of the properties cited in the above disclosure of the method of sulcus retraction and the dental kit.

The invention is explained in more detail by a non-limiting example and figures:
- Figures 1a - 1c: a simplified, schematic depiction of the method;
- Figure 2: a sectional drawing of a tooth with an applied retraction material and a dental dam.

### EXAMPLE:

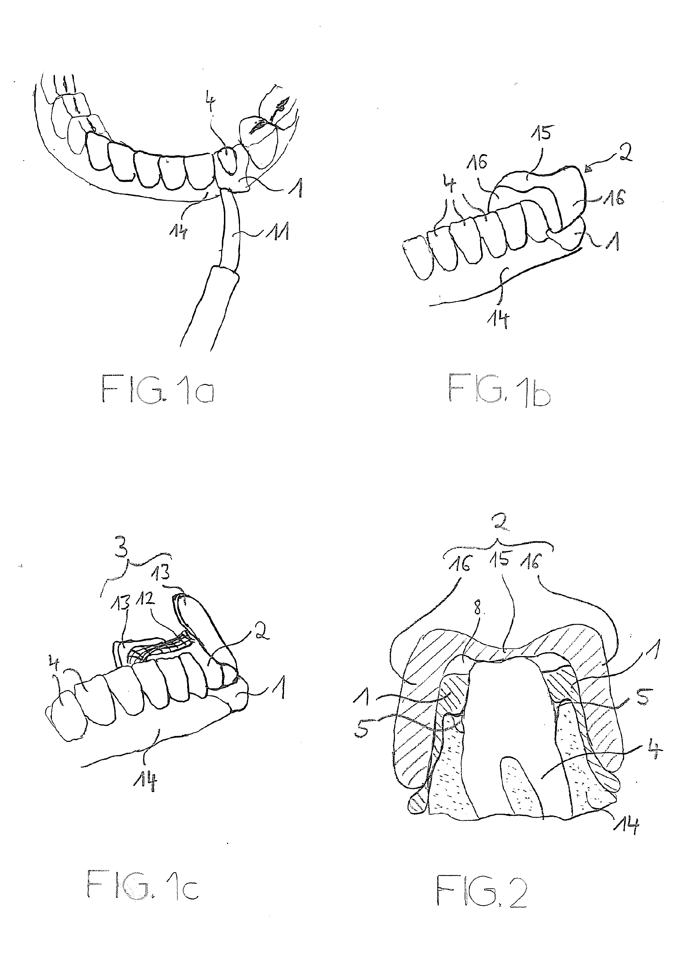

In a first step, which is depicted in Figure 1a, a retraction material 1 is applied to the sulcus of a tooth 4 by means of a nozzle 11. (For a sectional drawing showing the sulcus 5, see Figure 2.) The nozzle 11 is connected to a two-chamber cartridge system with a static mixing device, which is not shown here for simplicity. The retraction material 1 comprises two expanding silicone compounds consisting of two components A and B.

**Component A may consist of:**

| | |
|---|---|
| 10g | alpha, omega-dihydroxy-polydimethylsiloxan (viscosity 18 Pa.s, Wacker Silicones); |
| 5 g | milled quartz SIKRON B600 (Quarzwerke Frechen, D); |
| 0.05g | SILOPREN U-Katalysator Pt/D (GE Bayer Silicones); |
| 0.02g | Divinyltetramethyldisiloxan (Fluka). |

**Component B may consist of:**

| | |
|---|---|
| 9.08g | alpha, omega-Divinyl-polydimethylsiloxan (viscosity 20 Pa.s, Wacker Silicones); |
| 5g | milled quartz SIKRON B600 (Quarzwerke Frechen, D); |
| 0.3g | Polymethylhydrosiloxan (viscosity 20 mPa.s; Wacker Silicones). |

Both components A and B are homogeneously mixed at a mass ratio of 1:1. Due to the immediate mixing of the silicone components A and B during the application, the curing reaction is initiated.

Prior to the complete curing of the retraction material 1, a dental dam 2 is applied to the tooth 4, as shown in Figure 1b. The dental dam 2 is provided as a blank, i. e. in a preformed, but non-individualized shape. It possesses a top portion 15, which is located on or above the tooth 4, and two lateral portions 16, which are situated on either side of the tooth 4. The dental dam 2 is applied in such a way that it also covers at least the adjacent sulcus as well as a part of surrounding gingiva 14 or a portion of the retraction material 1 covering said gingiva 14. Moreover, the dental dam 2 is placed in such a way that it comes into contact with at least part of the retraction material 1.

The plastic deformability of the dental dam 2 facilitates the method of conforming it to the tooth 4, the sulcus, the gingiva 14 and possibly the retraction material 1. The dental dam 2 comprises a divinylpolydimethylsiloxane solid silicone. Moreover, due to its Mooney viscosity ML(1+4/23 °C) of about 100 - 200 MU, the dental dam 2 is not flowable, but remains in its position and shape. In particular, no further cap is required in this procedure.

Optionally, as shown in Figure 1c, a common bite tray or impression tray 3 may be applied to cover the dental dam 2. In this example, the bite tray 3 comprises a net 12, which is placed on or above the top portion 15 of the dental dam 2. Furthermore, the bite tray 3 contains two support plates 13, which are arranged at two opposite ends of the net 12 and which are placed adjacent to the lateral portions 16 of the dental dam 2.

When both the retraction material 1 and the dental dam 2 have been applied, the patient is asked to clench his/her teeth in order to force the dental dam 2 and the retraction material 1 towards the sulcus 5. The optional bite tray 3 may prevent a further undesirable deformation or even a destruction of the dental dam 3. During its curing reaction, the retraction material 1 expands, thereby entering into the sulcus.

When the curing of the retraction material 1 has substantially terminated, the dental dam 2 is removed. Preferably, the material of the retraction material 1 and the dental dam 2 are chosen such that the retraction material 1 can be jointly removed by gripping and pulling only the dental dam 2 without a need to touch the retraction material 1. In this state, the dental dam 2 and the retraction material 1 have been chemically crosslinked and form a dental retraction device according to the invention.

In Figure 2, there is shown a sectional drawing of a tooth 4, which is being subjected to the method according to the invention. The figure captures the moment, in which the dental dam 2 has been applied, but the expansion of the retraction material 1 has not been fully terminated yet. The tooth 4 is embedded in gingiva 14. The upper border of the gingiva 14 is separated from the tooth 4 by the sulcus 5. The retraction material 1 surrounds the neck of the tooth 4 and covers the sulcus 5. A dental dam 2 is placed on the tooth 4, wherein its top portion 15 rests on the tooth 4. The two lateral portions 16 are in contact with at least part of the retraction material 1 and a portion of the gingiva 14, whereby a chamber 8 is formed above the retraction material 1. The chamber 8 comprises as its walls a portion of the tooth 4, a portion of the dental dam 2, and the sulcus 5.

In a further, optional step, which is not shown here, the dental dam 2 may be conformed to the shape of the tooth 4 and the surrounding gingiva 14 after its application onto the tooth 4, thereby removing or reducing the chamber 8. This optional step is preferred when no impression tray or bite tray is used.

## Claims

1. A method of sulcus retraction, comprising the steps of:
- applying a flowable, preferably a curable retraction material (1) onto the sulcus (5) of at least one tooth (4) and/or applying the said retraction material (1) into a dental dam (2);
- applying the dental dam (2) onto said at least one tooth (4); and
- allowing said dental dam (2) to get in contact with said retraction material (1) if the retraction material (1) has been applied onto the sulcus (5); or allowing said retraction material (1) in the dental dam (2) to get in contact with said sulcus (5);
wherein said retraction material (1) and the material of said dental dam (2) form a chemical bond, preferably a covalent chemical bond, with each other.

2. Method according to claim 1, **characterized in that** said dental dam (2) is plastically deformable.

3. Method according to one of claims 1 and 2, **characterized in that** said dental dam (2) has a Mooney viscosity ML(1+4/23 °C) of 1 - 300 MU, preferably of 10 - 250 MU, most preferably of 50 - 200 MU.

4. Method a according to one of claims 1 to 3, **characterized in that** said dental dam (2) contains at least one silicone compound or a mixture of silicone compounds.

5. Method according to one of claims 1 to 4, **characterized in that** said retraction material (1) contains at least one silicone compound crosslinkable by an addition reaction.

6. Method according to one of claims 1 to 5, **characterized in that** said retraction material (1) is curable and can form a chemical bond with said dental dam (2) during and/or after curing.

7. Method according to one of claims 1 to 6, **characterized in that** said chemical bond is formed by reacting alkyl, alkenyl, alkynyl, hydroxyl, or hydride groups, thereby covalently crosslinking said dental dam (2) to said retraction material (1).

8. Method according to one of claims 1 to 7, **characterized in that** said retraction material (1) is an impression material.

9. Method according to one of claims 1 to 7, **characterized in that** said retraction material (1) expands, in particular during and/or after curing, thereby exhibiting a volume expansion of at least 20 %, preferably of at least 35 %, more preferably of at least 70 %.

10. Method according to one of claims 1 to 9, **characterized in that** the components for the production of said retraction material are provided in a multi-chamber cartridge system (6), preferably in a two-chamber cartridge system, and **in that** the application is performed via a mixing device (7), preferably by a static mixing device.

11. Method according to one of claims 1 to 10, **characterized in that** by applying said dental dam (2), a chamber (8) is formed over said retraction material (1), wherein said chamber (8) comprises as its walls at least a portion of said at least one tooth (4), at least a portion of said dental dam (2), and an outer section of the gingiva (14) or the retraction material (1) on said gingiva (14).

12. Method according to one of claims 1 to 11, comprising the step of covering the said dental dam (2) by an impression tray or bite tray (3).

13. Method according to one of claims 1 to 12, comprising the step of the further step of jointly removing said dental dam (2) and said retraction material (1) from said tooth (4) after the formation of said chemical bond.

14. Dental kit (9) for performing sulcus retraction, preferably for a method of sulcus retraction according to one of claims 1 to 13, containing at least one flowable, preferably a curable retraction material (1) and at least one dental dam (2),
wherein said retraction material (1) and said dental dam (2) are chosen such that they can form a chemical bond, preferably a covalent chemical bond, with each other.

15. Dental kit (9) according to claim 14, **characterized in that** at least one dental dam (2) is plastically deformable.

16. Dental kit (9) according to one of claims 14 and 15, **characterized in that** at least one dental dam (2) has a Mooney viscosity ML(1+4/23 °C) of 1 - 300 MU, preferably of 10 - 250 MU, most preferably of 50 - 200 MU.

17. Dental kit (9) according to one of claims 14 to 16, **characterized in that** at least one dental dam (2) contains at least one silicone compound or a mixture of silicone compounds.

18. Dental kit (9) according to one of claims 14 to 17, **characterized in that** the retraction material (1) comprises at least one silicone compound crosslinkable by an addition reaction.

19. Dental kit (9) according to one of claims 14 to 18, **characterized in that** said chemical bond between the dental dam (2) and the retraction material (1) has a higher tear-off force than the bond between the retraction material (1), preferably the cured retraction material, and a tooth (4) and/or or a portion of a gingiva (14).

20. Dental kit (9) according to one of claims 14 to 19, **characterized in that** it further contains at least one impression tray and/or at least one bite tray (3) for covering said dental dam (2).

21. Use of a dental kit (9) according to one of claims 14 to 20 for retraction of sulcus according to a method according to one of claims 1 to 13.

22. A dental retraction device for retracting a sulcus (5) comprising a retraction material (1), preferably a cured retraction material, and a dental dam (2), wherein said retraction material (1) and said dental dam (2) have formed a chemical bond with each other.

23. Dental retraction device according to claim 22, **characterized in that** said dental dam (2) is plastically deformable.

24. Dental retraction device according to one of claims 22 and 23, **characterized in that** said dental dam (2) has a Mooney viscosity ML(1+4/23 °C) of 1 - 300 MU, preferably of 10 - 250 MU, most preferably of 50 - 200 MU..

25. Dental retraction device according to one of claims 22 to 24, **characterized in that** said dental dam (2) contains at least one silicone compound or a mixture of silicone compounds.

26. Dental dam (2), **characterized in that** it has a Mooney viscosity ML(1+4/23 °C) of 1 - 300 MU, preferably of 10 - 250 MU, most preferably of 50 - 200 MU.
